## Europäisches Patentamt
## European Patent Office
## Office européen des brevets

(19)

(11) Numéro de publication: **0 265 409**
**A1**

(12)
# DEMANDE DE BREVET EUROPEEN

(21) Numéro de dépôt: **87870124.2**

(22) Date de dépôt: **14.09.87**

(51) Int. Cl.⁴: **C 12 M 1/12**
**C 12 P 7/56**

(30) Priorité: **15.09.86 LU 86584**

(43) Date de publication de la demande:
**27.04.88 Bulletin 88/17**

(84) Etats contractants désignés:
**AT BE CH DE ES FR GB GR IT LI NL SE**

(71) Demandeur: **SYNFINA - OLEOFINA S.A.**
**Zone Industrielle D**
**B-6538 Manage (BE)**

(72) Inventeur: **Hofman, Marcel**
**Hondsbergen 2**
**B-1981 Tervueren-Vossem (BE)**

(74) Mandataire: **Detrait, Jean-Claude**
**c/o LABOFINA S.A. Zone Industrielle C**
**B-6520 Feluy (BE)**

(54) **Procédé de préparation d'acide lactique par fermentation de lactoserum.**

(57) On décrit un procédé de production d'acide lactique par fermentation, au départ d'un perméat de lactosérum contenant du lactose à raison de 30-70 g/l. Le fermenteur (4) est muni d'un système d'agitation (5) et thermostatisé entre 28 et 45°C. On y introduit le perméat de lactosérum (1), des bactéries du type Lactobacillus à haute vitesse d'acidification, et des biofacteurs (6). Après ultrafiltration (9) du moût de fermentation, le rétentat est renvoyé au fermenteur (10) et le filtrat (11) est pompé dans un système de colonnes à échange d'ions (20). On récupère l'acide lactique par élution frontale avec une solution d'un acide minéral fort.

**Description**

## PROCEDE DE PREPARATION D'ACIDE LACTIQUE PAR FERMENTATION DE LACTOSERUM

La présente invention se rapporte à un procédé de préparation d'acide lactique par fermentation de lactosérum, en présence de bactéries lactiques. En particulier, la présente invention concerne un procédé pour préparer en continu l'acide lactique, ainsi qu'un appareillage pour réaliser ce procédé. Plus particulièrement encore, le procédé de la présente invention se rapporte à une production d'acide lactique par fermentation extractive de lactosérum.

On sait que le lactosérum est un sous-produit de faible valeur provenant de la transformation du lait pour produire des produits à haute valeur ajoutée comme le fromage. Il est donc intéressant de pouvoir valoriser le lactosérum notamment pour conduire à la formation d'autres produits par fermentation.

On a déjà pu obtenir à partir de lactosérum pris comme substrat de fermentation, du méthane, de l'éthanol ainsi que des levures à usages alimentaires. Cependant, le produit le plus intéressant à préparer reste l'acide lactique. Celui-ci est obtenu par fermentation du lactose contenu dans le lactosérum.

Cependant, les procédés de fermentation de lactose ont de nombreux inconvénients, du fait notamment de la nécessaire addition de neutralisant au milieu, ce qui est défavorable à l'élaboration d'un procédé en continu, et surtout aux opérations de purification qui résultent de la présence de lactate.

Ainsi, dans les procédés traditionnels, on utilise comme substrat de fermentation du lactosérum, de la mélasse... supplémentés par des produits appropriés pour obtenir une teneur convenable en protéine et vitamine. Les souches utilisées pour la fermentation sont du genre Lactobacillus et de l'un des groupes choisis parmi Thermobacterium et Streptobacterium. La fermentation est conduite en présence de neutralisant, en l'occurence le carbonate de calcium, qui neutralise l'acide lactique formé par fermentation au fur et à mesure de sa formation. Quand l'espèce de départ est épuisée, on porte à ébullition et on filtre. Le filtrat contient seulement 10 % d'acide lactique brut, qui doit être purifié pour être utilisable. Ce procédé conduit à une perte importante en matière de départ, mais surtout à un faible rendement.

Afin de remédier à certains inconvénients, on a déjà proposé d'utiliser d'autres souches, comme Lactobacillus LBR, à grande vitesse de production d'acide lactique, ou encore en essayant de retirer l'acide lactique du milieu au fur et à mesure de sa production.

Récemment, on a encore proposé de purifier le produit résultant de l'ultrafiltration par électrodialyse, mais encore une fois, ce type de procédé ne donne pas de rendements significativement plus élevés, mais de plus ne peut qu'être difficilement mené en continu puisqu'il y a toujours addition de neutralisant au milieu de fermentation.

La présente invention a pour objet un nouveau procédé pour produire de l'acide lactique par fermentation à partir de lactosérum, ce procédé ne nécessitant pas d'addition d'un quelconque neutralisant au milieu de fermentation.

La présente invention a également pour objet un procédé de production d'acide lactique par fermentation permettant d'obtenir un rendement en acide lactique nettement plus élevé.

La présente invention a également pour objet un procédé de production d'acide lactique par fermentation, qui permette d'éviter les nombreuses opérations de purification prévues dans les procédés antérieurs.

La présente invention a également pour objet un procédé de production d'acide lactique par fermentation qui puisse être conduit isément en continu, sans risque de blocage du réacteur de fermentation.

Le procédé de la présente invention pour produire de l'acide lactique par fermentation est caractérisé en ce qu'il comporte les étapes suivantes :
- on introduit dans un réservoir tampon un perméat de lactosérum contenant de 30 à 70 g de lactose/l;
- on pompe le lactosérum dans un fermenteur muni d'un système d'agitation et thermostatisé à une température précise comprise entre 28 et 45 °C;
- on introduit dans le fermenteur des bactéries du type Lactobacillus à haute vitesse d'acidification ainsi que des biofacteurs;
- on soumet le moût de fermentation à l'ultrafiltration;
- on renvoie le rétentat au fermenteur;
- on pompe le filtrat dans un système de colonnes à échange d'ions comprenant au moins une colonne échangeuse d'anions;
- on récupère l'acide lactique par élution frontal avec une solution d'un acide minéral fort.

Selon le procédé de l'invention, on introduit dans un fermenteur du lactosérum soit déprotéiné, soit tel quel. Le lactosérum peut ainsi être utilisé sans avoir à subir une décationisation préalable.

On réalise ensuite la fermentation généralement en présence de bactéries lactiques; lorsque l'on utilise ce type de bactéries, on emploie les lactobacilles thermophiles à haute vitesse d'acidification. Il faut remarquer que dans le procédé de l'invention, l'addition de neutralisant n'est pas requise pour le contrôle du pH, ce qui constitue un avantage certain.

On réalise la fermentation dans des conditions de culture telles qui l'on freine fortement la propagation d'autres microorganismes. Les conditions opératoires types sont les suivantes.

La température de fermentation est choisie entre 28 et 45 °C. Le fermenteur est muni d'un système de thermostat pour maintenir la température définie de fermentation.

La quantité de sucre règle l'alimentation au fermenteur, et on la stabilise dans les effluents à environ 3 à 5 g/l d'effluent.

Comme biofacteurs appropriés que l'on ajoute au milieu de fermentation, on peut notamment citer les extraits de levures, les bactopeptones, ou les hydrolysats protéiques comme les lysats de poisson. Afin de faciliter l'introduction de ces biofacteurs dans le milieu, il est souvent souhaitable d'ajouter un agent solubilisant, comme par exemple l'acétate de sodium, ou un autre agent approprié.

En même temps que les biofacteurs, on peut également ajouter au milieu un traceur.

Le moût de fermentation obtenu à l'étape précédente est soumis à une étape d'ultrafiltration qui permet de récupérer d'une part un rétentat contenant principalement des cellules entières ainsi que des produits de la lyse cellulaire, et d'autre part l'ultrafiltrat contenant l'acide lactique. Le rétentat est recyclé au moins partiellement au fermenteur, tandis que l'ultrafiltrat est envoyé pour traitement dans les colonnes échangeuses d'ions.

Le système de traitement de l'ultrafiltrat peut être à colonne unique ; dans ce cas, celle-ci est remplie d'une résine échangeuse d'anions, utilisée sous forme hydroxyle. Ctte résine capte l'acide lactique de fermentation, et l'effluent de la colonne est partiellement recyclé dans le fermenteur de façon à réguler le pH de ce dernier. Lorsque la colonne est saturée, on introduit l'effluent de l'ultrafiltration dans une autre colonne, tandis que l'on régénère la première par élution frontale avec un courant d'un acide fort, du moins plus fort que l'acide lactique, jusqu'à récupération complète de l'acide lactique. Cette colonne est ensuite régénérée à l'ammoniaque de manière à se retrouver sous sa forme hydroxyle initiale.

Selon un mode d'exécution préféré du procédé de l'invention, on utilise un système de traitement à deux colonnes échangeuses d'ions. On envoie l'effluent de l'ultrafiltration dans une première colonne remplie d'une résine échangeuse d'anions, utilisée sous forme hydroxyle, où se fixent les anions dont les ions lactates. Comme les anions d'acides forts sont préférentiellement fixés sur cette première colonne, celle-ci se sature en de tels ions et les ions lactates sont déplacés et fixés sur une deuxième colonne de même type. L'effluent de cette dernière colonne est partiellement recyclé au fermenteur pour les mêmes raisons que ci-dessus. Ce mode d'exécution offre l'avantage de permettre, en ajustant les conditions opératoires, d'obtenir une seconde colonne sur laquelle il n'y a pratiquement que des anions lactates.

Selon un mode d'exécution plus préféré, on place, en aval de la deuxième colonne, une troisième colonne du même type, ce qui permet der saturer la deuxième colonne tout en fixant la totalité des ions lactates.

Selon un mode d'exécution de l'invention, la résine présente dans la seconde colonne est également utilisée sous sa forme hydroxyle, mais il va de soi que d'autres contre-ions peuvent être utilisés, comme par exemple les ions acétates.

Lorsque cette seconde colonne est saturée, l'ion lactate fixé dans celle-ci est libéré sous forme d'acide lactique par élution frontale avec un courant d'une solution d'un acide fort, du moins plus fort que l'acide lactique, jusqu'à récupération complète de l'acide lactique.

Les deux colonnes sont ensuite régénérées avec de l'ammoniaque, ou, selon le cas, avec un autre composé comme un acétate, de manière à se retrouver sous leur forme initiale.

Selon un mode préféré de réalisation du procédé de l'invention, on fait préalablement passer l'effluent de l'ultrafiltration sur une colonne échangeuse de cations, ce qui a pour effet d'éliminer les cations de l'influent des résines échangeuses d'ions et ainsi de favoriser la fixation ultérieure de l'acide lactique.

Il est évident que ces différentes colonnes doivent fonctionner en alternance avec d'autres de manière à pouvoir assurer le fonctionnement continu de l'opération.

Il est souhaitable d'effectuer la récupération de l'acide lactique par élution frontale avec un courant ascendant d'une solution, plus dense que celle d'acide lactique, d'un acide minéral non oxydant plus fort que l'acide lactique.

A titre d'exemple d'un tel acide, on peut notamment citer l'acide sulfurique. Il est entendu que l'on pourrait déplacer l'acide lactique par un courant descendant d'une solution d'un acide fort, mais moins dense que celle d'acide lactique, par exemple une solution d'HCl.

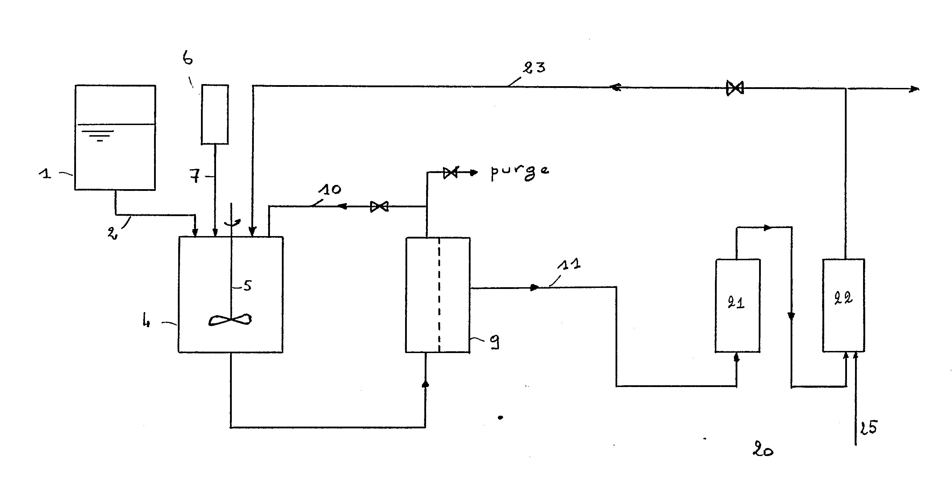

Le procédé de l'invention est également décrit à l'aide du dessin annexé dans lequel la Figure représente un schéma complet du procédé de l'invention.

En se référant à la Figure, on pompe en continu par la conduite 2 le lactosérum se trouvant dans le réservoir tampon 1 dans le fermenteur 4 équipé d'un système de chauffage et d'un agitateur (5).

On y ajoute par la conduite 7 les biofacteurs, éventuellement l'agent solubilisant, et si on le souhaite le traceur, contenus dans le réservoir 6.

On envoie le moût de fermentation, en continu, par la conduite 8 dans le dispositif d'ultrafiltration 9. Le rétentat de l'ultrafiltration est recyclé par la conduite 10 dans le fermenteur tandis que l'ultrafiltrat est envoyé par la conduite 11 dans le dispositif de colonnes échangeuses d'ions 20. On a représenté un système à deux colonnes.

L'ultrafiltrat est envoyé dans la première colonne 21 qui est remplie d'une résine échangeuse d'anions, utilisée sous sa forme hydroxyle. Cette première colonne se sature en anions d'acides forts, tandis que les ions lactates, qui s'y sont fixés au début, sont progressivement déplacés et vont se fixer dans la deuxième colonne 22 qui contient également une résine échangeuse d'anions utilisée sous sa forme hydroxyle. En cours de fonctionnement, l'effluent de cette colonne 22 est partiellement recyclé au fermenteur par la conduite 23 afin de réguler le pH de ce dernier. Lorsque la colonne 22 est saturée en ions lactates, on la lave d'abord à l'eau pour éliminer l'effluent de fermentation qui se trouve dans la colonne, puis on envoie par la conduite 25 un courant ascendant d'une solution d'acide fort qui, par élution frontale, déplace les ions lactates sous forme d'acide lactique. Lorsque cette opération est terminée, on régénère les colonnes 21 et 22 de manière à ce qu'elles se retrouvent sous leur forme initiale.

Les exemples suivants sont donnés afin de mieux illustrer le procédé de la présente invention mais sans pour autant en limiter la portée.

Exemple 1

On a réalisé une fermentation en continu pendant 10 jours.

On a alimenté un fermenteur de 20 l muni d'une sonde de pH, d'un système de régulation de température, et d'un système de contrôle de niveau, avec une solution de perméat de lactosérum à 70 g lactose/l.

On y a ajouté 75 g d'extrait de levure, 37 g d'acétate de sodium (utilisé comme agent solubilisant) et 0,4g de MnSO$_4$ (utilisé comme traceur) par 15 l de perméat. La température dans le fermenteur était de 40°C.

Le temps de séjour moyen dans le fermenteur était compris entre 25 et 40 heures. La densité optique s'est stabilisée à environ 30. On soutirait le moût de fermentation à raison de 0,2 à 0,6 l/min. et on l'envoyait à l'ultrafiltration.

Le système de colonnes échangeuses d'ions était alimenté par le liquide provenant de l'étape d'ultrafiltration, à un débit de 0,17 à 0,32 l/min.

Le système utilisé est un système à 2 colonnes fonctionnant en alternance. Ce sont des colonnes de 3 l remplies avec 2 l de résine Duolite A 374. Le milieu sortant de la colonne en fonctionnement est recyclé au fermenteur.

Lorsque la colonne est saturée en acide lactique, on introduit dans la colonne de l'acide sulfurique à contre-courant, de bas en haut, et à un débit de 30 ml/min., de manière à déplacer l'acide lactique et à le récupérer.

Dans l'essai qui a été réalisé, on récupère la fraction passant entre pH 1,6 et pH 1 et on obtient 600 ml d'acide lactique à 16 % par litre de résine.

Exemple 2

On a réalisé une fermentation en discontinu pendant 48 heures dans le fermenteur décrit à l'exemple 1. Le volume du milieu était de 15 l d'une solution de perméat de lactosérum à 70 g/l de lactose. La température était de 40°C.

Après 23 heures, on a ajouté 75 g de bactopeptones, 37 g d'acétate de Na et 0,37 g de MnSO$_4$.

Le moût de fermentation a été soumis à l'ultrafiltration et l'ultrafiltrat a été pompé dans le système de colonnes échangeuses d'ions décrit à l'exemple 1.

La colonne a été saturée après 18 heures de passage de l'ultrafiltrat, à un débit de 12 l/hr.

On a ainsi introduit de l'acide sulfurique à contre-courant et de bas en haut et à un débit de 30 ml/min de manière à déplacer l'acide lactique.

Dans l'essai réalisé, on récupère la fraction passant entre pH 1,6 et pH 1 et on obtient 560 ml d'acide lactique à 32 % par litre de résine.

**Revendications**

1) Procédé de production d'acide lactique par fermentation, caractérisé en ce qu'il comprend les étapes qui consistent à :

- introduire dans un réservoir tampon un perméat de lactosérum contenant de 30 à 70 g de lactose par litre;
- pomper le lactosérum dans un fermenteur muni d'un système d'agitation et thermostatisé à une température précise comprise entre 28 et 45°C;
- introduire dans le fermenteur des bactéries du type lactobacillus à haute vitesse d'acidification ainsi que des biofacteurs;
- soumettre le moût de fermentation à l'ultrafiltration;
- renvoyer le rétentat au fermenteur;
- pomper le filtrat dans un système de colonnes à échange d'ions comprenant au moins une colonne échangeuse d'anions;
- récupérer l'acide lactique par élution frontale avec une solution d'un acide minéral fort.

2) Procédé de production d'acide lactique selon la revendication 1, caractérisé en ce que l'on pompe le filtrat de l'ultrafiltration dans un système de colonnes d'échange d'ions comprenant au moins deux colonnes, et de préférence trois ou quatre colonnes.

3) Procédé de production d'acide lactique selon la revendication 2, caractérisé en ce que le système de colonnes échangeuses d'ions comprend au moins une première colonne pour le prétraitement et une seconde colonne échangeuse d'anions.

4) Procédé de production d'acide lactique selon la revendication 3, caractérisé en ce que le système de colonnes échangeuses d'ions comprend, pour le prétraitement, une colonne échangeuse de cations, suivie d'une colonne échangeuse d'anions pour retenir les anions fixés préférentiellement au lactate.

5) Procédé de production d'acide lactique selon la revendication 3, caractérisé en ce que le système de colonnes échangeuses d'ions comprend, pour fixer les ions lactates, deux colonnes échangeuses d'anions en série.

6) Procédé de production d'acide lactique selon l'une quelconque des revendications 1 à 5, caractérisé en ce que l'on utilise comme biofacteurs des extraits de levures, des bactopeptones et/ou des hydrolysats protéiques comme des lysats de poisson.

7) Procédé de production d'acide lactique selon l'une quelconque des revendications 1 à 6, caractérisé en ce que l'on réalise la récupération de l'acide lactique par élution frontale avec un acide minéral qui est plus fort que l'acide lactique, en solution plus dense que celle d'acide lactique.

8) Procédé de production d'acide lactique selon la revendication 7, caractérisé en ce que l'on utilise l'acide sulfurique pour récupérer l'acide lactique par élution frontale.

purge

1 · 2 · 4 · 5 · 6 · 7 · 9 · 10 · 11 · 20 · 21 · 22 · 23 · 15

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR-A-2 555 200  (C.N.R.S)<br>* Revendications 1,3; figure 1 *<br>--- | 1-4,7,8 | C 12 M    1/12<br>C 12 P    7/56 |
| Y | CHEMICAL ABSTRACTS, vol. 93, no. 13, 29 septembre 1980, page 515, abrégé no. 130609m, Columbus, Ohio, US; M.M. VOZLINSKII et al.: "Lactic acid", & SU-A-735 590 (ALL-UNION SCIENTIFIC-RESEARCH INSTITUTE OF MICROBIOLOGICAL PLANT-PROTECTING AG) 25-05-1980<br>* En entier *<br>--- | 1-4,7,8 | |
| A | FR-A-1 031 371  (VEREINIGTE MAUTNER MARKOF'SCHE PRESSHEFE FABRIKEN)<br>----- | | |

DOMAINES TECHNIQUES
RECHERCHES (Int. Cl.4)

C 12 M
C 12 P

Le présent rapport a été établi pour toutes les revendications

| Lieu de la recherche | Date d'achèvement de la recherche | Examinateur |
|---|---|---|
| LA HAYE | 27-11-1987 | WIESER, M.R.J. |

EPO FORM 1503 03.82 (P0402)